# EUROPEAN PATENT SPECIFICATION

(11) **EP 4 339 655 B1**
(45) Date of publication and mention of the grant of the patent: **04.02.2026**
(21) Application number: 22807123.9
(22) Date of filing: 04.03.2022
(51) Int. Cl.: G01W 1/10, G01W 1/14, G01W 1/08, G06N 3/08, G06N 20/00, G01N 22/00, G01N 33/18

(54) **GENERATION METHOD FOR LEARNING MODEL, COMPUTER PROGRAM, ESTIMATION METHOD, AND ESTIMATION DEVICE**
ERZEUGUNGSVERFAHREN FÜR LERNMODELL, COMPUTERPROGRAMM, SCHÄTZVERFAHREN UND SCHÄTZVORRICHTUNG
PROCÉDÉ DE GÉNÉRATION POUR MODÈLE D'APPRENTISSAGE, PROGRAMME INFORMATIQUE, PROCÉDÉ D'ESTIMATION ET DISPOSITIF D'ESTIMATION

(30) Priority: 11.05.2021 JP 2021080522
(43) Date of publication of application: 20.03.2024
(73) Proprietor: Furuno Electric Co., Ltd., Nishinomiya-City, Hyogo 662-8580 (JP)
(72) Inventor: IWAHORI, Taiki, Nishinomiya-City, Hyogo 662-8580 (JP); MINOWA, Masahiro, Nishinomiya-City, Hyogo 662-8580 (JP)
(74) Representative: Müller Hoffmann & Partner
(86) International application number: PCT/JP2022/009542
(87) International publication number: WO 2022/239416

(56) References cited:
- WO-A1-2020/230501
- WO-A1-2020/230501
- WO-A1-2022/014229
- CN-A- 111 060 992
- CN-A- 111 220 986
- CN-A- 111 638 565
- CN-A- 112 232 554
- CN-A- 112 462 369
- JP-A- 2010 060 444
- JP-A- 2019 211 342
- US-A1- 2011 218 734
- US-A1- 2021 041 299
- BEREZIN I A ET AL: "Comparison of ground-based microwave measurements of precipitable water vapor with radiosounding data", ATMOSPHERIC AND OCEANIC OPTICS, M A I K NAUKA - INTERPERIODICA, RU, vol. 29, no. 3, 15 June 2016 (2016-06-15), pages 274 - 281, XP035982129, ISSN: 1024-8560, [retrieved on 20160615], DOI: 10.1134/S1024856016030040

## Description

The disclosure relates to a generation method for a learning model, a computer program, an estimation method, and an estimation device.

It is known that a GNSS receiver, a microwave radiometer, and the like are used for observation of a precipitable water amount, that is, water vapor observation. Water vapor observation using a global navigation satellite system (GNSS) receiver uses radio waves of a plurality of frequencies radiated from satellites. When radio waves of two or more different frequencies radiated from four or more satellites can be received, it is possible to ascertain a delay of radio waves. The delay of radio waves corresponds to an amount of water vapor and enables observation of the amount of water vapor (for example, Patent Literature 1). A rainfall prediction system described in Patent Literature 1 calculates a precipitable water amount based on a zenith atmosphere delay calculated from received GPS (GNSS) data and orbit information of satellites. WO 2020/230501 A1 discloses a water observation device comprising a weather sensor for measuring atmospheric pressure and temperature, a GNSS receiver for measuring GNSS signals and altitude, a microwave radiometer for measuring radio wave intensities, and a processing module. A water vapor index calculated from radio wave intensities is correlated to a GNSS precipitable water based on atmospheric delay of GNSS signals, and the correlation data are used to calculate precipitable water amount from radio wave intensities.

[Patent Literature 1]
Japanese Patent Laid-Open No. 2010-60444

However, since the rainfall prediction system described in Patent Literature 1 calculates the precipitable water amount based on the GNSS data and requires orbit information of satellites, it is difficult to improve a temporal resolution for calculating the precipitable water amount. Altitude-by-altitude water vapor densities cannot be calculated from GNSS data.

The disclosure was made in consideration of the aforementioned circumstances and provides a generation method for a learning model or the like that can efficiently estimate altitude-by-altitude water vapor densities or a precipitable water amount.

### [Solution to Problem]

The above problem is solved by the subject-matter of the independent claims. Advantageous embodiments are described in the dependent claims.

According to the disclosure, it is possible to provide a generation method for a learning model or the like that can efficiently estimate altitude-by-altitude water vapor densities or a precipitable water amount.

FIG. 1 is a block diagram illustrating an example of a configuration of a microwave radiometer according to a first embodiment.
FIG. 2 is a functional block diagram illustrating functional units (at the time of learning) included in a control unit of the microwave radiometer.
FIG. 3 is an explanatory diagram illustrating an example of observation data from the microwave radiometer.
FIG. 4 is an explanatory diagram illustrating an example of a radio field intensity of microwaves (sky-bb) included in the observation data.
FIG. 5 is a diagram schematically illustrating an example of a method of generating a precipitable water amount model through machine learning.
FIG. 6 is a diagram schematically illustrating an example of a method of generating an altitudinal water vapor distribution model through machine learning.
FIG. 7 is a flowchart illustrating an example of a routine (at the time of learning) which is performed by the control unit of the microwave radiometer.
FIG. 8 is a functional block diagram illustrating functional units (at the time of operating) included in the control unit of the microwave radiometer.
FIG. 9 is an explanatory diagram (a precipitable water amount change graph) illustrating an example of a graphed precipitable water amount change information.
FIG. 10 is an explanatory diagram (an altitudinal water vapor distribution graph) illustrating an example of a graphed altitudinal water vapor distribution.
FIG. 11 is a flowchart illustrating an example of a routine (at the time of operating) which is performed by the control unit of the microwave radiometer.
FIG. 12 is a block diagram illustrating an example of a system configuration of a microwave radiometer according to a second embodiment (a plurality of spots).
FIG. 13 is a flowchart illustrating an example of a routine which is performed by a control unit of a center server.
FIG. 14 is an explanatory diagram illustrating an example of an observation state at a plurality of spots (superimposed in map information).

### (First embodiment)

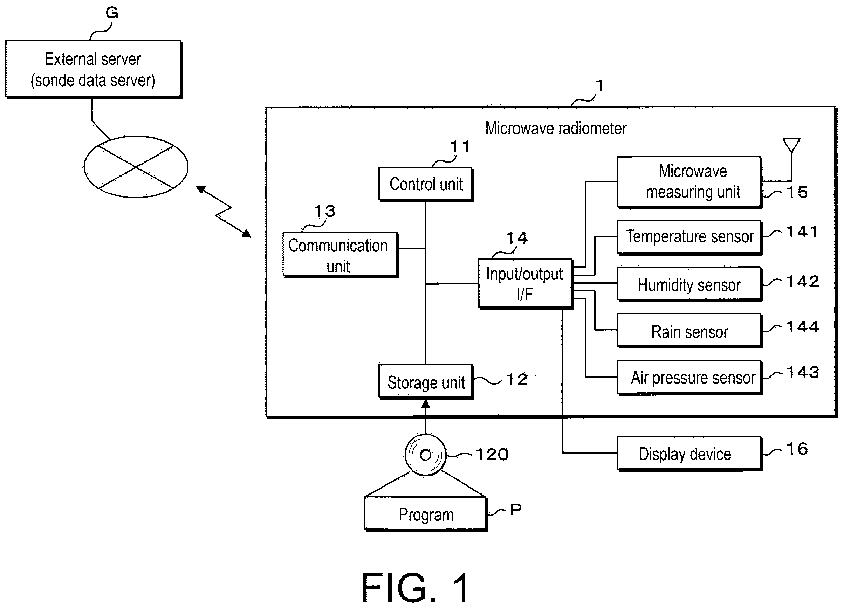

Hereinafter, an embodiment of the disclosure will be described. FIG. 1 is a block diagram showing an example of a configuration of a microwave radiometer 1 according to a first embodiment. The microwave radiometer 1 is communicatively connected to an external server G (a sonde data server) in which altitudinal temperature, humidity, and air pressure (sonde data) measured by a radiosonde are stored via an external network such as the Internet.

The microwave radiometer 1 generates training data based on sonde data such as altitude-by-altitude temperatures acquired from the external server G and radio field intensities of microwaves measured at an observation spot at which a radiosonde for observing the sonde data is released. The sonde data such as altitude-by-altitude temperatures acquired from the external server G corresponds to second measurement data, and the radio field intensities of microwaves measured by the microwave radiometer 1 at the observation spot at which the radiosonde is released corresponds to first measurement data.

Although details will be described later, the microwave radiometer 1 serves as a learning model generation device that trains, for example, parameters of a neural network using the generated training data and generating a precipitable water amount model 121, an altitudinal water vapor distribution model 122, or both models. The microwave radiometer 1 serves as a precipitable water amount estimation device (a water vapor observation device) that estimates a precipitable water amount in the sky in the vertical direction at a spot at which the microwave radiometer 1 is installed by inputting radio field intensities of microwaves measured at the spot to the precipitable water amount model 121. The microwave radiometer 1 serves as an altitudinal water vapor distribution estimation device (a water vapor observation device) that estimates altitude-by-altitude water vapor densities (an altitudinal water vapor distribution) in the sky in the vertical direction at the spot at which the microwave radiometer 1 is installed by inputting the radio field intensities of microwaves measured at the spot to the generated altitudinal water vapor distribution model 122.

The microwave radiometer 1 generates training data for generating a learning model (the precipitable water amount model 121 or the altitudinal water vapor distribution model 122) based on the measured radio field intensities of microwaves, but is not limited thereto. The microwave radiometer 1 may generate the learning model using training data which is generated based on temperature, humidity, air pressure, and rainfall at the spot at which the microwaves have been measured in addition to the radio field intensities of microwaves.

The microwave radiometer 1 generates input data for generating a learning model (the precipitable water amount model 121 or the altitudinal water vapor distribution model 122) based on the measured radio field intensities of microwaves, but is not limited thereto. The microwave radiometer 1 may acquire a precipitable water amount or an altitudinal water vapor distribution by inputting the input data which is generated based on temperature, humidity, air pressure, and rainfall at the spot at which the microwaves have been measured to the learning model (the precipitable water amount model 121 or the altitudinal water vapor distribution model 122) in addition to the radio field intensities of microwaves.

The external server G is constituted by, for example, a cloud server connected to the Internet or the like and is managed by a public institution (the Japan Meteorological Agency) that operates radiosondes. In the external server G, sonde data such as altitudinal temperature, humidity (relative humidity), and air pressure measured (observed) by the radiosondes released at observation spots is stored in correlation with the observation spots and the observation date and time. The sonde data can be acquired by accessing the external server G.

The microwave radiometer 1 includes a microwave measuring unit 15 configured to measure microwaves radiated from the atmosphere and a temperature sensor 141, a humidity sensor 142, an air pressure sensor 143, and a rain sensor 144 for acquiring climatological data at the spot at which the microwave radiometer 1 is installed. The microwave radiometer 1 further includes a control unit 11, a storage unit 12, a communication unit 13, and an input/output I/F 14 for processing or handling various types of measurement data acquired by the sensor group.

The microwave measuring unit 15 includes, for example, a radio wave window, a black body, a receiving horn, and an AD conversion circuit, receives microwaves input through the radio wave window using the receiving horn, and converts the received microwaves to radio field intensities (dB) for frequencies using the AD conversion circuit. In this embodiment, for example, microwaves of about 40 channels (frequencies) in the range of from 16 GHz to 26 GHz are measured, and a radio field intensity (dB) of each of the 40 channels is measured. The peak of the radio field intensities of radio waves radiated from water vapor and cloud water in the sky appears at 22 GHz, and it is possible to improve identification accuracy of a water vapor component and a cloud water component by acquiring (measuring) the radio field intensities of the about 40 channels (frequencies) in the range of from 16 GHz to 26 GHz.

The microwave radiometer 1 alternately acquires microwaves radiated from a black body and microwaves radiated from the sky by periodically causing the black body to pass through the reception range of the receiving horn using a motor or the like and periodically positioning the black body to cover the receiving horn with respect to the sky. The black body is constituted, for example, by vantablack and corresponds to a reference radio wave absorber. In this way, the microwave radiometer 1 repeatedly performs a process of measuring microwaves from the reference radio wave absorber (the black body), for example, for 10 seconds and then measuring microwaves from the sky, for example, for 10 seconds. The microwave radiometer 1 acquires a difference value (sky-bb [dB]) obtained by subtracting the radio field intensity (bb [dB]) of the microwaves from the black body from the radio field intensity (sky [dB]) of the microwaves from the sky as the radio field intensity of the measured microwaves for each of the 40 channels (frequencies).

In measuring (observing) microwaves from the black body and the sky, the microwave radiometer 1 may automatically calibrate linearity of analog instruments or the like (detector calibration). The microwave radiometer 1 may switch an attenuator connected to the receiving horn, ascertain whether a digital value obtained by conversion in the AD conversion circuit is linear with respect to a pre-conversion analog value (both are in a proportional relationship based on an ideal straight line), and perform linearity calibration of an analog system when the digital value obtained by AD conversion exceeds an allowable error range.

The temperature sensor 141 is constituted, for example, by a thermistor and detects air temperature at the spot at which the microwave radiometer 1 is installed. The humidity sensor 142 is, for example, a capacitance-change or resistance-change electric sensor, detects an amount of water vapor in the ambient air of the spot at which the microwave radiometer 1 is installed, and converts the detected amount to an electrical signal. The air pressure sensor 143 is constituted, for example, by a pressure receiving device and detects air pressure (atmospheric pressure) at the spot at which the microwave radiometer 1 is installed. The rain sensor 144 is, for example, a sensor that linearly changes a voltage value which is output based on an amount of rainfall, detects an amount of rainfall at the spot at which the microwave radiometer 1 is installed, and converts the detected amount to an electrical signal.

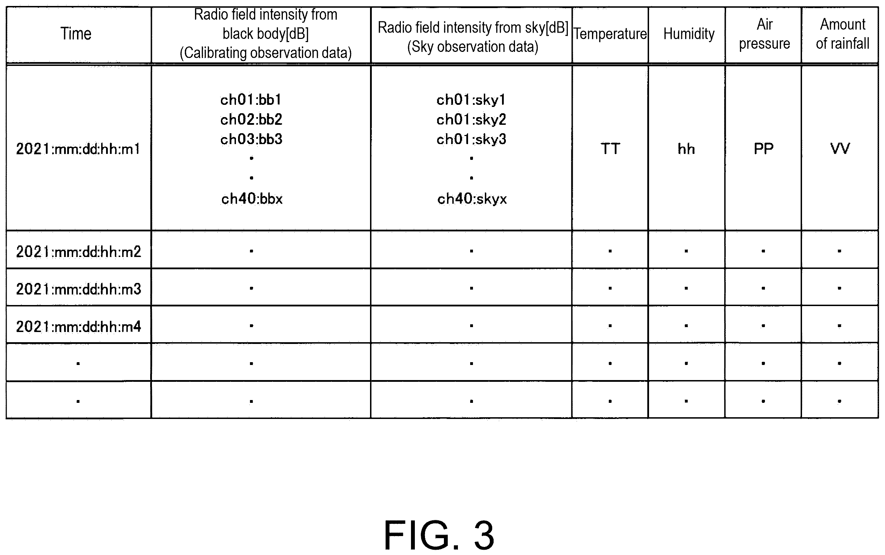

The microwave measuring unit 15, the temperature sensor 141, the humidity sensor 142, the air pressure sensor 143, and the rain sensor 144 are communicatively connected to the control unit 11 and the storage unit 12, for example, via the input/output I/F 14 or an internal bus. Measurement data measured by the microwave measuring unit 15, the temperature sensor 141, and the like is correlated with time information indicating a time point at which it is measured and is stored, for example, as time-series data in a table form (see FIG. 3) in the storage unit 12.

The control unit 11 includes an arithmetic processing unit having a timer function such as one or more central processing units (CPUs), micro-processing units (MPUs), or graphics processing units (GPUs) and performs various information processes, a control process on the microwave measuring unit 15, and the like by reading and executing a program P stored in the storage unit 12.

The storage unit 12 includes a volatile storage area such as a static random access memory (SRAM), a dynamic random access memory (DRAM), or a flash memory and a nonvolatile storage area such as an EEPROM or a hard disk. The program P and data which is referred to at the time of processing are stored in the storage unit 12 in advance. The program P stored in the storage unit 12 may be a program P (a program product) which is read from a recording medium 120 readable by the control unit 11 and which is stored therein. A program P (a program product) may be downloaded from an external computer (not illustrated) connected to a communication network which is not illustrated and stored in the storage unit 12. Actual files constituting learning models (the precipitable water amount model 121 and the altitudinal water vapor distribution model 122) are stored in the storage unit 12. These actual files may constitute a part of the program P.

The communication unit 13 is a communication module or a communication interface for communicating with an external server G (a sonde data server) in a wired or wireless manner and includes, for example, a local-area radio communication module such as Wi-Fi (registered trademark) or Bluetooth (registered trademark) or a wide-area radio communication module such as 4G or 5G. The control unit 11 communicates with the external server G via an external network such as the Internet using the communication unit 13.

The input/output I/F 14 includes, for example, a communication interface based on a communication protocol such as USB or a connector for connection to the internal bus. The microwave measuring unit 15, the temperature sensor 141, the humidity sensor 142, the air pressure sensor 143, and the rain sensor 144 may be connected to the input/output I/F 14. A display device 16 such as a liquid crystal display may be additionally connected to the input/output I/F 14.

In this embodiment, the microwave radiometer 1 includes the microwave measuring unit 15, various sensors, and the control unit 11 for performing data processing, but is not limited thereto. The control unit 11 or the like for performing data processing may be provided in a computer separate from the microwave radiometer 1, the microwave radiometer 1 and the computer may be communicatively connected, and the computer may perform generation of learning models and estimation using the learning models by processing various types of data measured by the microwave radiometer 1. The computer may acquire (receive) observation data (radio field intensities and climatological data) from microwave radiometers 1 installed at a plurality of observation spots which are observed by radiosondes and generate training data based on the observation data and the sonde data for each observation spot. The learning models (a precipitable water amount model and an altitudinal water vapor distribution model) having learned the observation data and the sonde data at the plurality of observation spots in this way may be applied to the individual microwave radiometers 1, and the individual microwave radiometers 1 may perform an estimation process using the learning models.

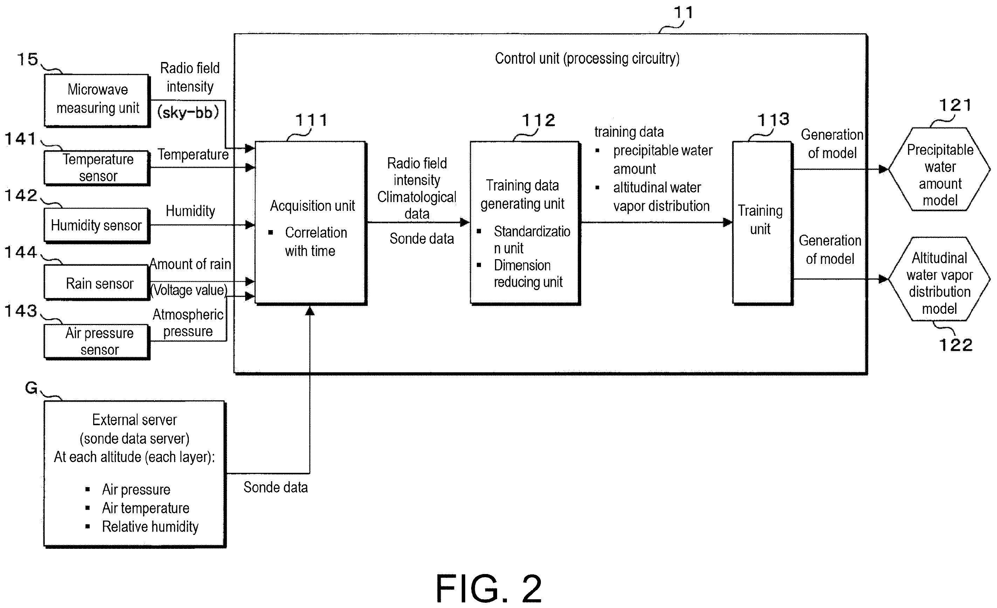

FIG. 2 is a functional block diagram illustrating functional units (at the time of learning) included in the control unit 11 of the microwave radiometer 1. The control unit 11 of the microwave radiometer 1 serves as an acquisition unit 111, a training data generating unit 112, and a training unit 113 by executing a program P stored in the storage unit 12.

The acquisition unit 111 acquires, for example, radio field intensities (first measurement data) in each of about 40 channels (frequencies) in the range of from 16 GHz to 26 GHz from the microwave measuring unit 15. The acquisition unit 111 acquires measured values (climatological data) such as temperature output from the temperature sensor 141, the humidity sensor 142, the air pressure sensor 143, and the rain sensor 144. The acquisition unit 111 acquires the radio field intensities (first measurement data) and the measured values (climatological data) such as temperature, the microwave radiometer 1 is installed in a spot (area) in which observation by a radiosonde is performed. A time period in which the acquisition unit 111 acquires the radio field intensities (first measurement data) and the measured values (climatological data) is the same as a time period in which observation by the radiosonde is performed. That is, the radio field intensities (first measurement data) and the measured values (climatological data) such as temperature acquired by the acquisition unit 111 geographically and temporally correspond to sonde data observed (measured) by the radiosonde.

FIG. 3 is an explanatory diagram illustrating an example of observation data from the microwave radiometer 1. The acquisition unit 111 correlates the acquired radio field intensities (first measurement data) and the measured values (climatological data) such as temperature with a time point (a measurement time point) at which the data has been acquired and stores the correlated data, for example, in a table format (an observation data table) in the storage unit 12. The observation data table includes time, a radio field intensity from a black body, a radio field intensity from the sky, temperature, humidity, air pressure, and rainfall as management items (fields).

In the management item (field) of time, date and time information indicating a date and time indicating of an observation time point (an acquisition time point) of observation data (radio field intensities and climatological data) is stored. In the management item (field) of a radio field intensity from a black body, radio field intensities of about 40 channels (frequencies) in the range of from 16 GHz to 26 GHz radiated from the black body are stored as calibrating observation data. In the management item (field) of radio field intensity from the sky, radio field intensities of about 40 channels (frequencies) in the range of from 16 GHz to 26 GHz radiated from the sky are stored as sky observation data.

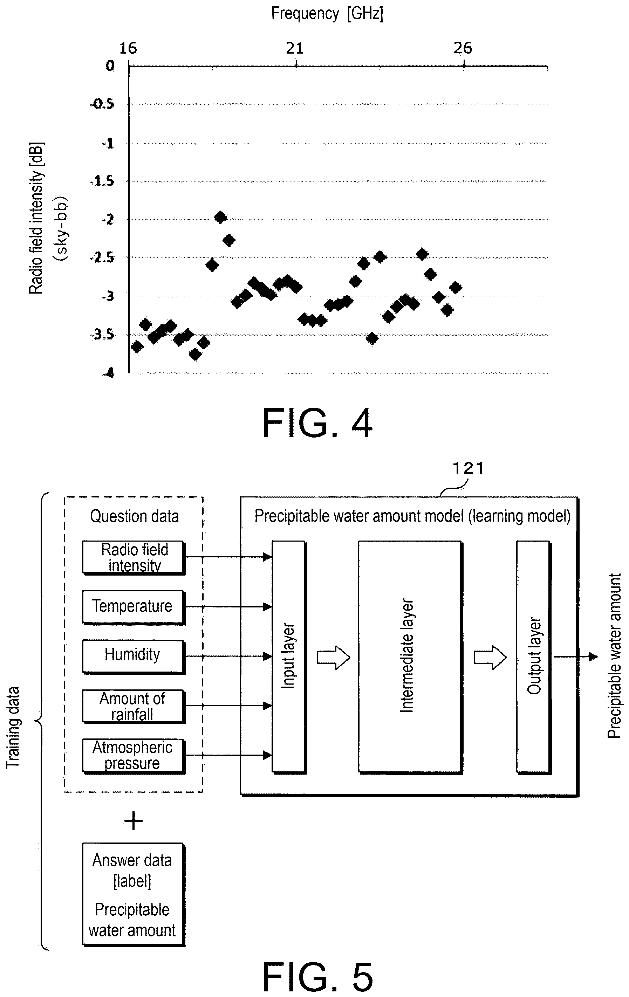

In this embodiment, the radio field intensities from the black body and the sky are stored in a table format, but is not limited thereto. A difference value (sky-bb) obtained by subtracting the radio field intensity (bb) of the black body from the radio field intensity (sky) of the sky may be stored. FIG. 4 is an explanatory diagram illustrating an example of the radio field intensity of microwaves (sky-bb) included in the observation data. In the explanatory diagram, the vertical axis represents the radio field intensity of the difference value (sky-bb) and the horizontal axis represents a channel (frequency). Since the black body (the reference radio wave absorber) is at the normal temperature (high temperature) and has a higher radiation intensity than that of the sky, the difference value (sky-bb) obtained by subtracting the radio field intensity (bb) of the black body from the radio field intensity (sky) of the sky has, for example, a negative value of from 0 dB to about -5 dB.

In the management item (field) of temperature, the air temperature at the spot at which the microwave radiometer 1 is installed is stored. In the management item (field) of humidity, absolute humidity or relative humidity at the spot at which the microwave radiometer 1 is installed is stored. In the management item (field) of air pressure, the air pressure (atmospheric pressure) at the spot at which the microwave radiometer 1 is installed is stored. In the management item (field) of time, a voltage value indicating an amount of rainfall at the spot at which the microwave radiometer 1 is installed is stored.

The acquisition unit 111 acquires altitude-by-altitude temperatures, humidity, and air pressure (sonde data) measured by the radiosonde from the external server G. As described above, the sonde data and the observation data (the radio field intensities and the climatological data) geographically and temporally correspond to each other.

The training data generating unit 112 generates training data for generating (training) the precipitable water amount model 121 and the altitudinal water vapor distribution model 122 based on the observation data (the radio field intensities and the climatological data) measured by the microwave radiometer 1 and the sonde data acquired from the external server G. The training data includes question data generated based on the observation data (the radio field intensities and the climatological data) and answer data generated based on the sonde data. The radio field intensity is expressed as the difference value (sky-bb) obtained by subtracting the radio field intensity (bb) of the black body from the radio field intensity (sky) of the sky.

The observation data (the radio field intensities and the climatological data) measured by the microwave radiometer 1 temporally corresponds to the sonde data. The training data generating unit 112 may generate question data based on a plurality of pieces of observation data (the radio field intensities and the climatological data) measured at a plurality of time points included in a time period in which the sonde data is observed (measured). The training data generating unit 112 may generate question data based on averaged, standardized, or normalized observation data (radio field intensities and climatological data) by performing an averaging process, a standardization process, or a normalization process on the observation data (the radio field intensities and the climatological data) at the plurality of time points. That is, the training data generating unit 112 may include a standardization unit for standardizing observation data or the like.

The training data generating unit 112 may perform a dimension reducing process on first measurement data including, for example, the radio field intensities of about 40 channels (frequencies) in the range of from 16 GHz to 26 GHz and generate question data based on the first measurement data subjected to the dimension reducing process. That is, the training data generating unit 112 may include a dimension reducing unit that performs the dimension reducing process on the observation data. The dimension reducing process may perform the dimension reduction, for example, using principal component analysis (PCA) on the radio field intensities at a plurality of frequencies and use a particular number of principal components subsequent to the first rank as the first measurement data for generating (training) a learning model. The dimension reducing process may set the particular number of principal components to be, for example, 3 and select a first principal component, a second principal component, and a third principal component. The principal component analysis is not limited to PCA, and may use an algorithm such as factor analysis, multiple factor analysis, autoencoder, independent component analysis, or non-negative matrix factor analysis.

In this embodiment, the training data generating unit 112 generates question data based on the radio field intensities and the climatological data (temperature, humidity, air pressure, and rainfall) measured by the microwave radiometer 1, but is not limited thereto. The training data generating unit 112 may generate question data based on only the radio field intensity measured by the microwave radiometer 1. Alternatively, the training data generating unit 112 may generate question data based on temperature, humidity, and air pressure in addition to the radio field intensity. That is, the training data generating unit 112 may generate question data based on some data included in the climatological data in addition to the radio field intensity.

The training data generating unit 112 calculates altitude-by-altitude water vapor density by differentiating a total amount of water vapor (1/g*((r[i]+r[i+1])/2*(P[i]-P[i+1])))*0.1, "g=9.81 m/s^2, r=mixing ratio, P=air pressure") calculated using a known method in the altitudinal direction and calculates a precipitable water amount by integrating the altitude-by-altitude water vapor densities in the altitudinal direction. The altitude-by-altitude water vapor densities (altitudinal water vapor distribution) corresponds to answer data for generating (training) the altitudinal water vapor distribution model 122. The precipitable water amount corresponds to answer data for generating (training) the precipitable water amount model 121. The question data generated based on the observation data (the radio field intensities and the climatological data) is common data (question data) in generating (training) the precipitable water amount model 121 and the altitudinal water vapor distribution model 122.

The training data generating unit 112 generates training data for the altitudinal water vapor distribution model 122 for generating (training) the altitudinal water vapor distribution model 122 using the question data and the answer data including the altitude-by-altitude water vapor densities (altitudinal water vapor distribution) generated based on the sonde data. The training data generating unit 112 generates training data for the precipitable water amount model 121 for generating (training) the precipitable water amount model 121 using the question data and the answer data including the precipitable water amount generated based on the sonde data.

FIG. 5 is a diagram schematically illustrating an example of a method of generating the precipitable water amount model 121 using machine learning. The training unit 113 trains a neural network using the training data for the precipitable water amount model 121 and generates the precipitable water amount model 121 with the observation data (the radio field intensities and the climatological data) as an input and with the precipitable water amount as an output. The neural network (the precipitable water amount model 121) trained using the training data is assumed to be used as a program P module which is a part of artificial intelligence software. The precipitable water amount model 121 is used by the microwave radiometer 1 including the control unit 11 (such as the CUP) and the storage unit 12 as described above and is performed in the microwave radiometer 1 having such arithmetic processing capability to constitute a neural network system.

The precipitable water amount model 121 is constituted, for example, by a deep neural network (DNN) and includes an input layer for receiving an input of observation data, an intermediate layer for extracting feature quantities of the observation data, and an output layer for outputting a precipitable water amount. The input layer includes a plurality of neurons for receiving an input of values such as radio field intensities or temperature included in the observation data and delivers the input values to the intermediate layer. The intermediate layer is defined using an activation function such as an ReLu function or a sigmoid function, includes a plurality of neurons for extracting feature quantities of the input values, and delivers the extracted feature quantities to the output layer. Parameters such as weighting factors and bias values in the activation function are optimized using an error back-propagation method. The output layer is constituted, for example, by a fully coupled layer and outputs a precipitable water amount based on the feature quantities output from the intermediate layer.

In this embodiment, the precipitable water amount model 121 is a DNN, but is not limited thereto and it may be a learning model constructed using another learning algorithm of a neural network other than the DNN, such as a recurrent neural network (RNN), a long-short term model (LSTM), a CNN, a support vector machine (SVM), a Bayesian network, linear regression, a regression tree, multiple regression, random forest, or ensemble. For example, when multiple regression including a plurality of types of variables is used, the precipitable water amount model 121 may be expressed by a transformation using polynomial regression. In this case, the transformation is defined by an inner product of vectors between the same number of unknown coefficients (S1 to Sn) as the number of dimensions (n) of observation data (the radio field intensities and the climatological data) subjected to principal component analysis and the observation data subjected to principal component analysis, and these coefficients (S1 to Sn) are calculated, for example, by fitting using a least square method.

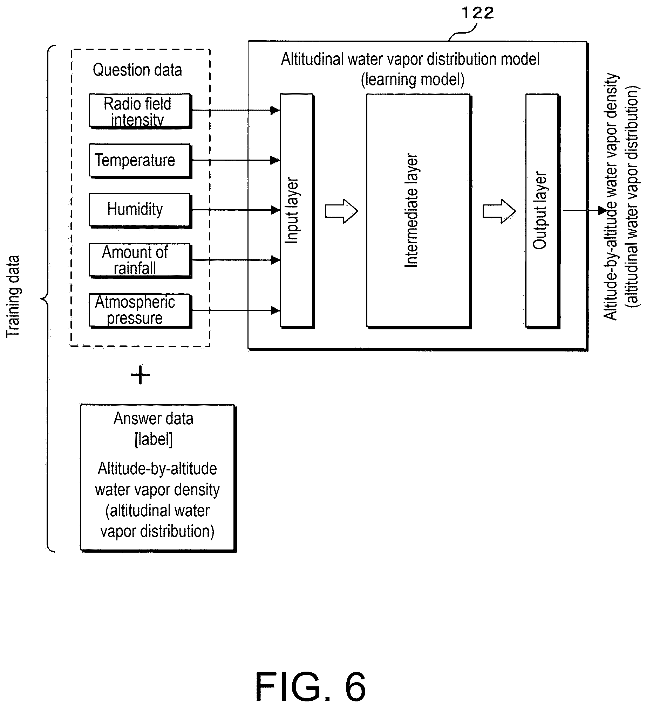

FIG. 6 is a diagram schematically illustrating an example of a method of generating the altitudinal water vapor distribution model 122 through machine learning. The training unit 113 trains a neural network using the training data for an altitudinal water vapor distribution model 122 and generates an altitudinal water vapor distribution model 122 with the observation data (the radio field intensities and the climatological data) as an input and with the altitudinal water vapor densities (the altitudinal water vapor distribution) as an output.

The altitudinal water vapor distribution model 122 is constituted, for example, by a DNN and includes an input layer for receiving an input of observation data, an intermediate layer for extracting feature quantities of the observation data, and an output layer for outputting an altitudinal water vapor distribution. Alternatively, the altitudinal water vapor distribution model 122 may be expressed by a transformation using polynomial regression using multiple regression including a plurality of types of variables. In this way, the altitudinal water vapor distribution model 122 has the same configuration as the precipitable water amount model 121, and the training unit 113 generates a altitudinal water vapor distribution model 122 in the same way as the precipitable water amount model 121.

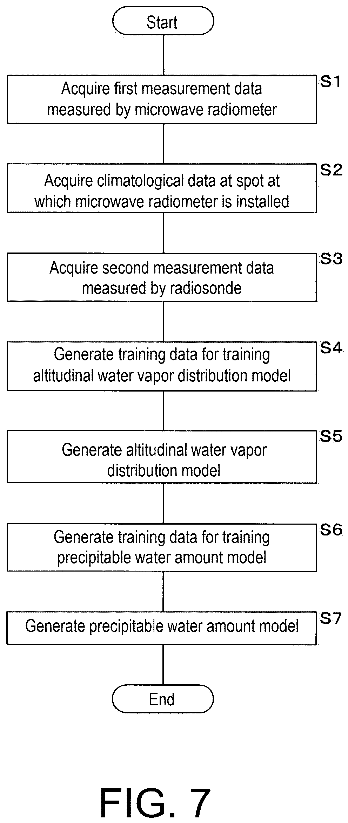

FIG. 7 is a flowchart illustrating an example of a routine (at the time of learning) that is performed by the control unit 11 of the microwave radiometer 1. For example, the control unit 11 of the microwave radiometer 1 receives an operation from an operator via a keyboard connected to the input/output I/F 14 and performs the following processes based on the received operation.

The control unit 11 of the microwave radiometer 1 acquires first measurement data measured by the microwave radiometer 1 (the microwave measuring unit 15) (S1). For example, the control unit 11 of the microwave radiometer 1 acquires radio field intensities (first measurement data) in about 40 channels (frequencies) in the range of from 16 GHz to 26 GHz. The control unit 11 of the microwave radiometer 1 may use a difference value (sky-bb) obtained by subtracting a radio field intensity (bb) of a black body from a radio field intensity (sky) of the sky in each of the channels (frequencies) as the first measurement data.

The control unit 11 of the microwave radiometer 1 acquires climatological data of a spot at which the microwave radiometer 1 is installed (S2). The control unit 11 of the microwave radiometer 1 acquires measured values (climatological data) such as temperature or the like output from the temperature sensor 141, the humidity sensor 142, the air pressure sensor 143, and the rain sensor 144. The radio field intensities (first measurement data) and the measured values (climatological data) such as temperature geographically and temporally correspond to sonde data measured by a radiosonde. The acquisition of climatological data is not limited to acquisition from the sensors such as the temperature sensor 141 provided in the microwave radiometer 1, and the control unit 11 of the microwave radiometer 1 may acquire climatological data of the spot at which the microwave radiometer 1 is installed, for example, from a climatological data server connected to the Internet or the like via the communication unit 13.

The control unit 11 of the microwave radiometer 1 acquires second measurement data measured by the radiosonde (S3). The control unit 11 of the microwave radiometer 1 accesses the external server G (the sonde data server) via the communication unit 13 and acquires sonde data (second measurement data) including altitude-by-altitude temperatures, humidity, and air pressure measured by the radiosonde.

The control unit 11 of the microwave radiometer 1 generates training data for training the altitudinal water vapor distribution model 122 (a learning model) that outputs altitude-by-altitude water vapor densities based on the first measurement data, the climatological data, and the second measurement data (S4). The control unit 11 of the microwave radiometer 1 generates training data for the altitudinal water vapor distribution model 122 based on the observation data (the radio field intensities and the climatological data) measured by the microwave radiometer 1 and the sonde data acquired from the external server G. In generating the training data, the control unit 11 of the microwave radiometer 1 may perform a standardization process or a dimension reducing process on the observation data (the radio field intensities and the climatological data) at a plurality of time points.

The control unit 11 of the microwave radiometer 1 generates the altitudinal water vapor distribution model 122 (a learning model) by performing training using the training data (S5). The control unit 11 of the microwave radiometer 1 generates the altitudinal water vapor distribution model 122 (a learning model), for example, by training a neural network using the training data for the altitudinal water vapor distribution model 122.

The control unit 11 of the microwave radiometer 1 generates training data for training the precipitable water amount model 121 (a learning model) that outputs a precipitable water amount based on the first measurement data, the climatological data, and the second measurement data (S6). The control unit 11 of the microwave radiometer 1 generates training data for the precipitable water amount model 121 based on the observation data (the radio field intensities and the climatological data) measured by the microwave radiometer 1 and the sonde data acquired from the external server G. In generating the training data, the control unit 11 of the microwave radiometer 1 may perform a standardization process or a dimension reducing process on the observation data (the radio field intensities and the climatological data) at a plurality of time points.

The control unit 11 of the microwave radiometer 1 generates the precipitable water amount model 121 (a learning model) by performing training using the training data (S7). The control unit 11 of the microwave radiometer 1 generates the precipitable water amount model 121 (a learning model), for example, by training a neural network using the training data for the precipitable water amount model 121.

In this embodiment, the control unit 11 of the microwave radiometer 1 acquires the radio field intensities (first measurement data) and the climatological data as the observation data, but is not limited thereto. The control unit 11 of the microwave radiometer 1 may generate training data based on only the radio field intensities (the first measurement) or some of the climatological data such as temperature, humidity, and air pressure in addition to the radio field intensities (the first measurement data) and generate the precipitable water amount model 121 and the altitudinal water vapor distribution model 122.

After generating the learning models (the precipitable water amount model 121 and the altitudinal water vapor distribution model 122), the control unit 11 of the microwave radiometer 1 may re-acquire training data including the first measurement data and the second measurement data and re-train the learning models generated already based on the re-acquired training data. Accordingly, it is possible to further improve estimation accuracy of the learning models (the precipitable water amount model 121 and the altitudinal water vapor distribution model 122) Re-training of the learning models (the precipitable water amount model 121 and the altitudinal water vapor distribution model 122) is not limited to fine tuning or transfer learning of a learning model generated already, and the learning models may be re-generated by training non-trained neural network using training data in which the re-acquired training data is added to the previously used training data. The control unit 11 of the microwave radiometer 1 may re-training the generated learning model in operating the generated learning model. In this case, the control unit 11 of the microwave radiometer 1 may derive a difference between a precipitable water amount estimated using the learning model and a precipitable water amount calculated based on the sonde data and re-train the learning model when the difference is greater than a particular value.

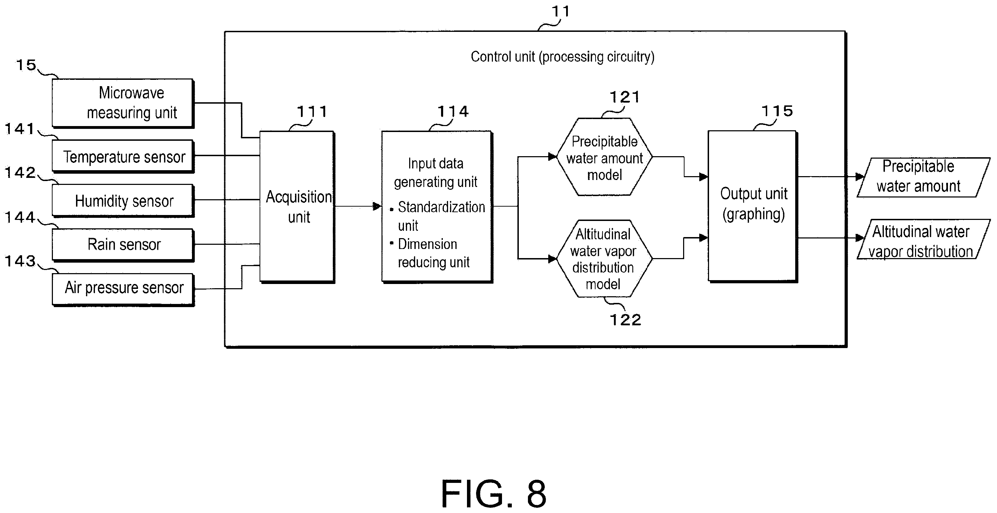

FIG. 8 is a functional block diagram illustrating functional units (at the time of operating) included in the control unit 11 of the microwave radiometer 1. The control unit 11 of the microwave radiometer 1 serves as an acquisition unit 111, an input data generating unit 114, and an output unit 115 by executing a program P stored in the storage unit 12.

The acquisition unit 111 acquires, for example, a radio field intensity (first measurement data) in each of about 40 channels (frequencies) in the range of from 16 GHz to 26 GHz from the microwave measuring unit 15. The acquisition unit 111 acquires measured values (climatological data) such as temperature or the like output from the temperature sensor 141, the humidity sensor 142, the air pressure sensor 143, and the rain sensor 144. The observation data including the radio field intensities and the climatological data acquired by the acquisition unit 111 is correlated with time points (measurement time points) at which the data has been acquired and stored, for example, in a table format (an observation data table) in the storage unit 12.

The input data generating unit 114 generates input data to be input to the precipitable water amount model 121 and the altitudinal water vapor distribution model 122 based on the observation data acquired by the acquisition unit 111. The radio field intensity included in the input data may be expressed as a difference value (sky-bb) obtained by subtracting the radio field intensity (bb) of the black body from the radio field intensity (sky) of the sky.

The input data generating unit 114 may generate input data based on a plurality of pieces of observation data measured at a plurality of time points in a particular time period. Similarly to the training data generating unit 112, the input data generating unit 114 may generate input data by performing a standardization process or a dimension reducing process on the observation data at the plurality of time points.

The input data generating unit 114 inputs the generated input data to the precipitable water amount model 121 and the altitudinal water vapor distribution model 122. In this way, the input data input to the precipitable water amount model 121 and the altitudinal water vapor distribution model 122 may be common.

The precipitable water amount model 121 estimates a precipitable water amount based on the input data input thereto. The altitudinal water vapor distribution model 122 estimates altitude-by-altitude water vapor densities (altitudinal water vapor distribution) based on the input data input thereto.

The output unit 115 converts the precipitable water amount estimated by the precipitable water amount model 121 and the altitude-by-altitude water vapor densities (altitudinal water vapor distribution) estimated by the altitudinal water vapor distribution model 122 to, for example, image data in the form of a graph and outputs the image data to the display device 16. The output unit 115 may convert the precipitable water amounts and the altitude-by-altitude water vapor densities (altitudinal water vapor distributions) at a plurality of successive observation time points to image data in the form of a graph indicating temporal change.

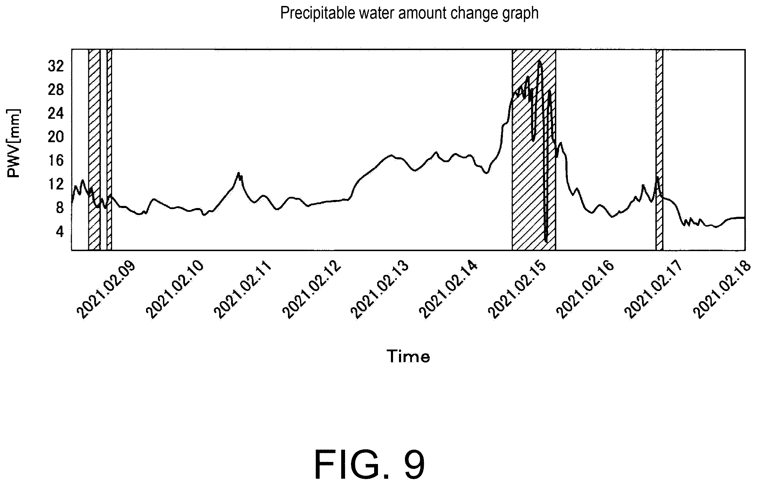

FIG. 9 is an explanatory diagram (a precipitable water amount change graph) illustrating an example of change information of graphed precipitable water amounts. The precipitable water amount change graph is an example of a graph indicating temporal change of the precipitable water amount at a plurality of successive observation time points. The horizontal axis of the precipitable water amount change graph represents the observation time point using the microwave radiometer 1. The vertical axis represents a precipitable water amount (PWV) in the sky (the atmosphere in the vertical direction) at the observation spot using the microwave radiometer 1.

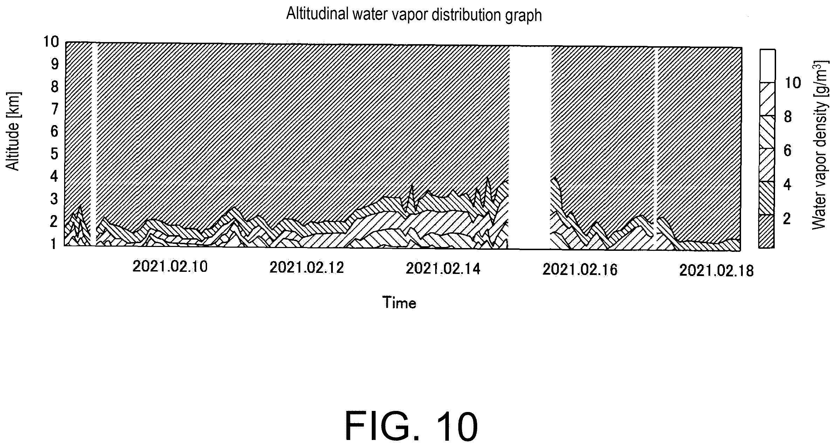

FIG. 10 is an explanatory diagram (a altitudinal water vapor distribution graph) illustrating an example of a graphed altitudinal water vapor distribution. The altitudinal water vapor distribution graph is an example of a graph indicating temporal change of the altitude-by-altitude water vapor densities (altitudinal water vapor distribution) at a plurality of successive observation time points. The horizontal axis of the altitudinal water vapor distribution graph represents the observation time point using the microwave radiometer 1. The vertical axis represents the altitude of the sky (the atmosphere in the vertical direction) at the observation spot using the microwave radiometer 1. The water vapor density is indicated by a contour diagram in which gradations of color tones vary according to the density.

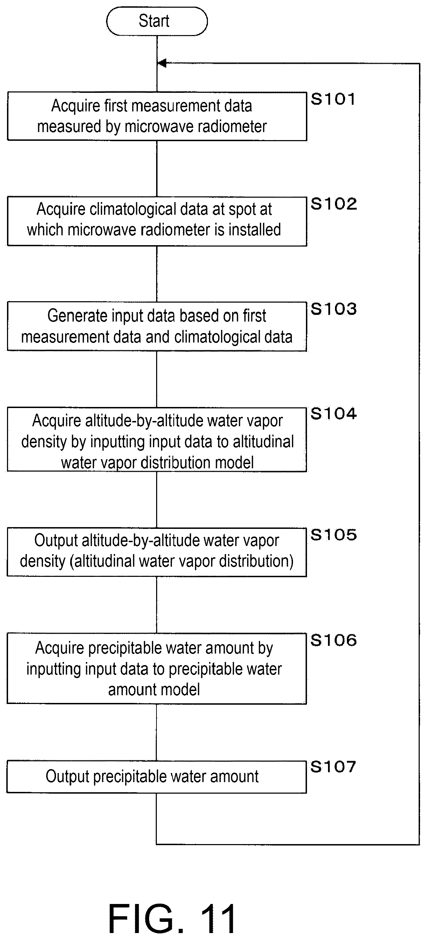

FIG. 11 is a flowchart illustrating an example of a routine (at the time of operating) that is performed by the control unit 11 of the microwave radiometer 1. For example, the control unit 11 of the microwave radiometer 1 receives an operation from an operator via a keyboard connected to the input/output I/F 14 and performs the following processes based on the received operation.

The control unit 11 of the microwave radiometer 1 acquires first measurement data measured by the microwave radiometer 1 (the microwave measuring unit 15) (S101). For example, the control unit 11 of the microwave radiometer 1 acquires climatological data at a spot at which the microwave radiometer 1 is installed (S102). Similarly to the processes of S1 and S2, the control unit 11 of the microwave radiometer 1 performs the processes of S101 and S102.

The control unit 11 of the microwave radiometer 1 generates input data based on the first measurement data and the climatological data (S103). The radio field intensity included in the input data may be expressed as a difference value (sky-bb) obtained by subtracting the radio field intensity (bb) of the black body from the radio field intensity (sky) of the sky. The control unit 11 of the microwave radiometer 1 may generate the input data based on a plurality of pieces of measurement data measured at a plurality of time points in a particular time period. The control unit 11 of the microwave radiometer 1 may generate the input data by performing a standardization process or a dimension reducing process on the observation data at the plurality of time points.

The control unit 11 of the microwave radiometer 1 inputs the input data to the altitudinal water vapor distribution model 122 and acquires altitude-by-altitude water vapor densities (altitudinal water vapor distribution) (S104). The control unit 11 of the microwave radiometer 1 inputs the input data to the altitudinal water vapor distribution model 122 and acquires the altitude-by-altitude water vapor densities (altitudinal water vapor distribution) estimated by the altitudinal water vapor distribution model 122.

The control unit 11 of the microwave radiometer 1 outputs the altitude-by-altitude water vapor densities (altitudinal water vapor distribution) (S105). For example, the control unit 11 of the microwave radiometer 1 graphs information on the altitude-by-altitude water vapor densities (altitudinal water vapor distribution) and outputs the graphed information to the display device 16.

The control unit 11 of the microwave radiometer 1 inputs the input data to the precipitable water amount model 121 and acquires a precipitable water amount (S106). The control unit 11 of the microwave radiometer 1 inputs the input data to the precipitable water amount model 121 and acquires the precipitable water amount estimated by the precipitable water amount model 121.

The control unit 11 of the microwave radiometer 1 outputs the precipitable water amount (S107). For example, the control unit 11 of the microwave radiometer 1 graphs information on the precipitable water amount and outputs the graphed information to the display device 16.

The control unit 11 of the microwave radiometer 1 performs a loop process to perform the routine from S101 again after the process of S107 has been performed. By repeating the processes from S101 to S107 in this way, it is possible to add or update and continue to output information on the precipitable water amount and the altitudinal water vapor distribution changing in a time series based on the successive observation results from the microwave radiometer 1. The control unit 11 of the microwave radiometer 1 may periodically update the graph displayed on the display device 16 by continuing to graph information on the precipitable water amount and the altitudinal water vapor distribution changing in a time series and to output the graphed information to the display device 16. That is, by sequentially inputting a plurality of pieces of input data generated from a plurality of pieces of observation data (the first measurement data and the climatological data) to the learning models (the precipitable water amount model 121 and the altitudinal water vapor distribution model 122), the control unit 11 of the microwave radiometer 1 can acquire the (a plurality of time-series) precipitable water amounts and the (a plurality of time-series) altitudinal water vapor distributions at a plurality of time points.

In this embodiment, the control unit 11 of the microwave radiometer 1 acquires the radio field intensities (first measurement data) and the climatological data in generating the input data, but is not limited thereto. The control unit 11 of the microwave radiometer 1 may generate input data based on only the radio field intensities (first measurement data) or some of the climatological data such as temperature, humidity, and air pressure in addition to the radio field intensities (first measurement data) and acquire a precipitable water amount and an altitudinal water vapor distribution by inputting the input data to the precipitable water amount model 121 and the altitudinal water vapor distribution model 122.

In this embodiment, training data for training the learning models (precipitable water amount model 121 and the altitudinal water vapor distribution model 122) uses the first measurement data measured by the microwave radiometer 1 as question data and the altitude-by-altitude water vapor densities or the precipitable water amount acquired from the second measurement data measured by the radiosonde as answer data. Accordingly, it is possible to efficiently generate a learning model trained using the second measurement data measured by the radiosonde and to efficiently output the altitude-by-altitude water vapor densities or the precipitable water amount based on the first measurement data measured by the microwave radiometer 1 using the learning model. It is possible to improve measurement accuracy of the first measurement data by using a difference value between a measured value of microwaves from the sky and a measured value of microwaves from a reference radio wave absorber such as a black body as the first measurement data and to improve estimation accuracy of the learning model by using the training data including the first measurement data.

In this embodiment, a plurality of frequencies included in the first measurement data is, for example, about 40 channels (frequencies) in the range of from 16 GHz to 26 GHz, and the radio field intensities (dB) of the 40 channels are stored in the storage unit 12. The control unit 11 can reduce the number of dimensions of the fist measurement data (question data) included in the training data by performing the dimension reducing process on the acquired and stored radio field intensities of the 40 channels, and thus it is possible to achieve a decrease in calculation cost.

In this embodiment, the question data included in the training data includes climatological data at the spot at which the microwave radiometer 1 is installed in addition to the first measurement data measured by the microwave radiometer 1. The climatological data may include at least one of temperature, humidity (absolute humidity or relative humidity), air pressure, and rainfall and may include all the physical quantities. By performing training using the question data, it is possible to improve estimation accuracy of the learning model.

### (Second embodiment)

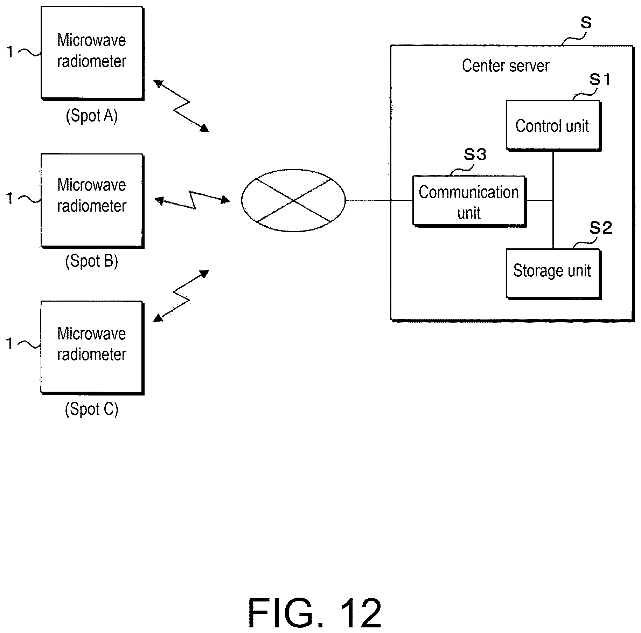

FIG. 12 is a block diagram illustrating an example of a system configuration of a microwave radiometer 1 according to a second embodiment (at a plurality of spots). In the second embodiment, a water vapor observation system is constituted by a plurality of microwave radiometers 1 installed at different spots and a center server S communicatively connected to the plurality of microwave radiometers 1.

The microwave radiometer 1 according to the second embodiment outputs measured observation data (radio field intensities and climatological data) to the center server S. The center server S is, for example, an information processing device such as a cloud server connected to the Internet and serves as an observation data collection server including a control unit S1, a storage unit S2, and a communication unit S3 and collecting observation data measured (observed) by the plurality of microwave radiometers 1 installed at different spots.

In the storage unit S2 of the center server S, the same precipitable water amount model 121 and the same altitudinal water vapor distribution model 122 as in the first embodiment are stored. Similarly to the control unit 11 of the microwave radiometer 1, the control unit S1 of the center server S generates input data for each spot based on the observation data output (transmitted) from the plurality of microwave radiometer 1 installed at different spots. The control unit S1 of the center server S outputs a precipitable water amount and an altitudinal water vapor distribution for each spot by inputting the generated input data for each spot to the precipitable water amount model 121 and the altitudinal water vapor distribution model 122.

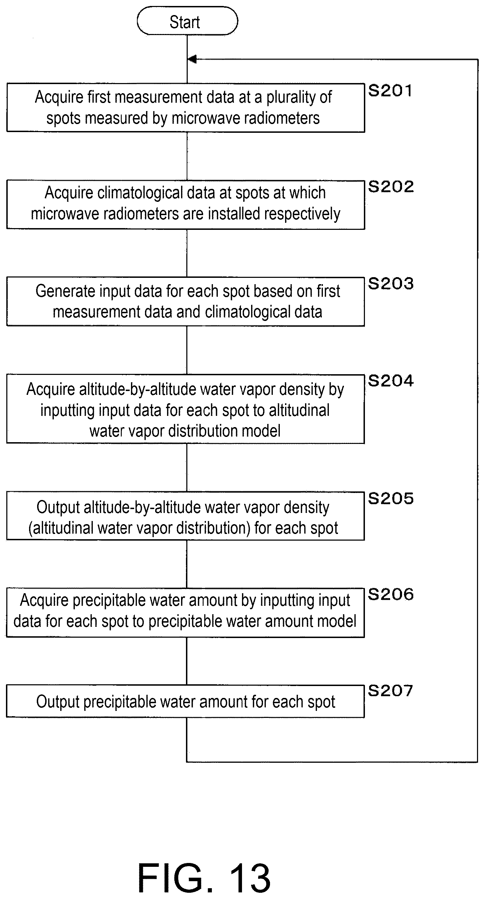

FIG. 13 is a flowchart illustrating an example of a routine that is performed by the control unit S1 of the center server S. The center server S performs the following processes normally, for example, when the server starts.

The control unit S1 of the center server S acquires first measurement data at a plurality of spots measured by the microwave radiometers 1 (S201). The control unit S1 of the center server S acquires radio field intensities (first measurement data) at a plurality of spots measured by the microwave radiometers 1 from the microwave radiometers 1 installed at the plurality of spots. Each radio field intensity may be a difference value (sky-bb [dB]) obtained by subtracting the radio field intensity (bb [dB]) of the black body from the radio field intensity (sky [dB]) of the sky.

The control unit S1 of the center server S acquires climatological data at the plurality of spots at which the microwave radiometers 1 are installed (S202). The control unit S1 of the center server S acquires climatological data including temperature, humidity, air pressure, and rainfall at the plurality of spots at which the microwave radiometers 1 are installed from the microwave radiometers 1 installed at the plurality of spots.

The control unit S1 of the center server S generates input data for each spot based on the first measurement data and the climatological data (S203). The control unit S1 of the center server S generates the input data for each spot based on the first measurement data and the climatological data for each spot (for each microwave radiometer 1) similarly to S103.

The control unit S1 of the center server S inputs the input data for each spot to the altitudinal water vapor distribution model 122 and acquires altitude-by-altitude water vapor densities (S204). The control unit S1 of the center server S outputs the altitude-by-altitude water vapor densities (altitudinal water vapor distribution) for each spot (S205). The control unit S1 of the center server S inputs the input data for each spot to the precipitable water amount model 121 and acquires a precipitable water amount (S206). The control unit S1 of the center server S outputs the precipitable water amount for each spot (S207). The control unit S1 of the center server S performs the processes of S204 to S207 on the input data for each spot (for each microwave radiometer 1) similarly to the processes of S104 to S107. Similarly to the first embodiment, the control unit S1 of the center server S performs a loop process to perform the routine from S201 again after the process of S207 has been performed.

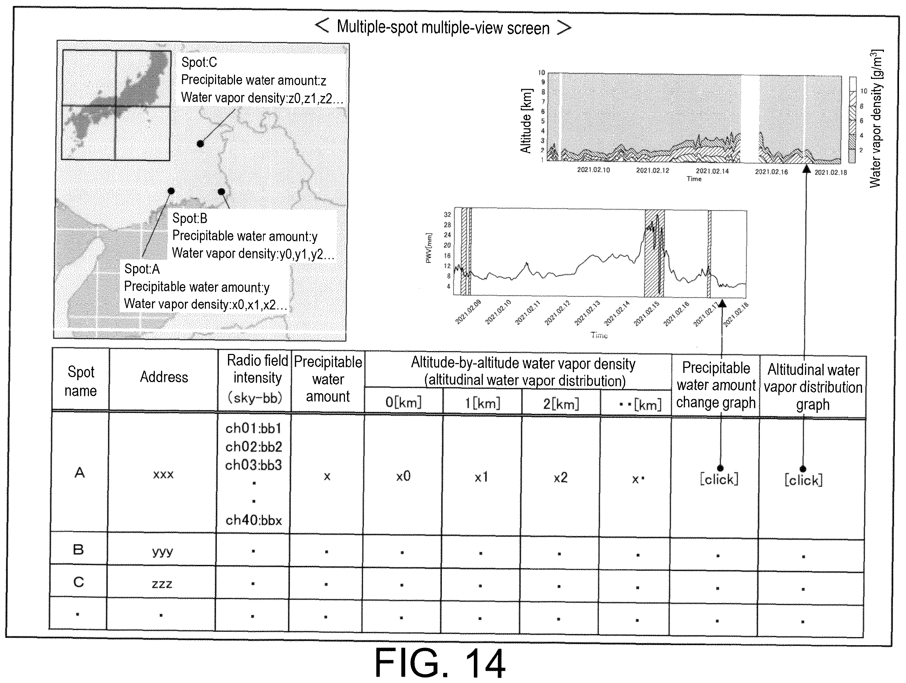

FIG. 14 is an explanatory diagram illustrating an example of an observation state (superimposed in map information) at a plurality of spots. The explanatory diagram (a multiple-spot multiple-view screen) is an example of screen data output from the center server S. The center server S acquires map information for identifying a plurality of spots at which a plurality of microwave radiometer 1 is installed, causes the altitude-by-altitude water vapor densities or the precipitable water amount at each of the plurality of spots on the acquired map information, and outputs the resultant information to the display device 16, a mobile terminal, or the like.

The multiple-spot multiple-view screen includes a map area in which the map information for identifying the spots at which the plurality of microwave radiometers 1 is installed and a list display area in which observation data measured (observed) by the microwave radiometers 1 is displayed in the form of a list. In the map area, the spots at which the microwave radiometers 1 are installed are displayed by a diagram, and the altitude-by-altitude water vapor densities and the precipitable water amount measured at each spot are superimposed and displayed. In the list display area, a list of radio field intensities and altitude-by-altitude water vapor densities measured at the spots is displayed. In the list display area, a button, an icon, or the like for which a hyperlink is set may be disposed, and a child screen for displaying a precipitable water amount change graph or an altitudinal water vapor distribution graph at a selected spot may be displayed in a pop-up manner by clicking the button or the like. By clicking the button or the like, a child screen for displaying a graph (a radio field intensity change graph) indicating temporal change of the radio field intensity (sky-bb) at the selected spot may be displayed in a pop-up manner.

In this embodiment, the control unit S1 of the center server S acquires observation data (first measurement data and climatological data) for each spot measured at each of a plurality of spots at which the microwave radiometers 1 are installed by radio communication, inputs the observation data for each spot to the learning models (the altitudinal water vapor distribution model 122 and the precipitable water amount model 121), and outputs altitude-by-altitude water vapor densities (altitudinal water vapor distribution) or a precipitable water amount for each of the plurality of spots. Since the control unit S1 of the center server S provides the altitude-by-altitude water vapor densities (altitudinal water vapor distribution) or the precipitable water amount for each spot in the form of a table, a graph, or the like for the purpose of enabling comparison and outputs the information to, for example, a mobile terminal via the communication unit S3, it is possible to efficiently provide the information to a weather relevant person or the like.

### [Reference Signs List]

G External server (sonde data server)
1 Microwave radiometer
11 Control unit
111 Acquisition unit
112 Training data generating unit
113 Training unit
114 Input data generating unit
115 Output unit
12 Storage unit
120 Recording medium
121 Precipitable water amount model
122 Altitudinal water vapor distribution model
P Program
13 Communication unit
14 Input/output I/F
141 Temperature sensor
142 Humidity sensor
143 Air pressure sensor
144 Rain sensor
15 Microwave measuring unit
16 Display unit
S Center server
S1 Control unit
S2 Storage unit
S3 Communication unit

## Claims

1. A generation method that is perfomed by a computer for generating a learning model (121, 122), comprising:
acquiring training data as question data from observation data measured by a microwave radiometer (1), the question data including altitude-by-altitude water vapor densities or a precipitable water vapor, and as answer data radiosonde data measured by a radiosonde, the answer data including altitude-by-altitude water vapor densities or a precipitable water vapor, wherein the observation data and the radiosonde data geographically and temporally correspond to each other; and
generating a trained model (121, 122) which outputs the altitude-by-altitude water vapor densities or the precipitable water amount based on the training data when question data is input.

2. A program (P) causing a computer to execute processing comprising:
acquiring observation data measured by a microwave radiometer (1); and
outputting altitude-by-altitude water vapor densities or a precipitable water vapor by inputting the observation data to the trained model (121, 122) generated by the method of claim 1, which outputs the altitude-by-altitude water vapor densities or the precipitable water vapor amount when the observation data is input as question data.

3. The program (P) according to claim 2, wherein the observation data includes a radio wave intensity for each of a plurality of frequencies.

4. The program (P) according to claim 3, wherein a dimension reduction process is performed on the radio wave intensity for each of the plurality of frequencies included in the observation data, and
the observation data including the radio wave intensity subjected to the dimension reduction process is input to the trained model (121, 122).

5. The program (P) according to any one of claims 2 to 4, wherein the training data used when generating the trained model further includes climatological data including temperature, humidity, air pressure, or rainfall at a spot at which the microwave radiometer (1) is installed, and the processing further comprising:
outputting the altitude-by-altitude water vapor densities or the precipitable water amount by inputting the observation data and climatological data to the trained model (121, 122).

6. The program (P) according to any one of claims 2 to 5, wherein a plurality of pieces of the observation data measured at a plurality of time points by the microwave radiometer (1) are acquired; and the processing further comprising:
generating change information of an altitudinal water vapor distribution based on the respective altitude-by-altitude water vapor densities at the plurality of time points output from the trained model (121, 122); and
outputting the change information of the altitudinal water vapor distribution.

7. The program (P) according to any one of claims 2 to 6, wherein a plurality of pieces of the observation data measured at a plurality of time points by the microwave radiometer (1) are acquired; and the processing further comprising:
generating change information of a precipitable water vapor based on the precipitable water vapor at the plurality of time points output from the trained model (121, 122); and
outputting the change information of the precipitable water vapor.

8. The program (P) according to any one of claims 2 to 7, wherein observation data for each spot measured by the respective microwave radiometers (1) installed in a plurality of spots is acquired; and the processing further comprising:
outputting the altitude-by-altitude water vapor densities or the precipitable water vapor at each of the plurality of spots by inputting the observation data for each spot to the trained model (121, 122).

9. The program (P) according to claim 8, wherein map information for identifying the plurality of spots at which the plurality of microwave radiometers (1) is installed is acquired; and the processing further comprising:
superimposing the altitude-by-altitude water vapor densities or the precipitable water vapor at each of the plurality of spots in the map information.

10. The program (P) according to claim 8 or 9, wherein climatological data including temperature, humidity, air pressure, or rainfall at each of the plurality of spots at which the plurality of microwave radiometers (1) is installed is acquired; and the processing further comprising:
inputting the observation data and the climatological data for each of the plurality of spots to the trained model (121, 122).

11. An estimation device comprising:
an acquisition unit (111) configured to acquire observation data measured by a microwave radiometer (1); and
an estimation unit configured to input the observation data to a trained model (121, 122) generated by the method of claim 1, and which outputs altitude-by-altitude water vapor densities or a precipitable water vapor when the observation is input as question data,
and estimate the altitude-by-altitude water vapor densities or the precipitable water vapor.

12. An estimation method that is performed by a computer, the estimation method comprising:
acquiring observation data measured by a microwave radiometer (1); and
inputting the observation data as question data to a learning model (121, 122) which has been generated by the method of claim 1 and which outputs altitude-by-altitude water vapor densities or a precipitable water vapor when the observation data is input, and estimating the altitude-by-altitude water vapor densities or the precipitable water vapor.

## Patentansprüche

1. Erzeugungsverfahren, das von einem Computer zum Erzeugen eines Lernmodells (121, 122) durchgeführt wird, aufweisend:
Erfassen von Trainingsdaten als Fragedaten aus Beobachtungsdaten, die von einem Mikrowellenradiometer (1) gemessen werden, wobei die Fragedaten Höhe-für-Höhe-Wasserdampfdichten oder einen niederschlagsfähigen Wasserdampf enthalten, und als Antwortdaten von Radiosondendaten, die von einer Radiosonde gemessen werden, wobei die Antwortdaten Höhe-für-Höhe-Wasserdampfdichten oder einen niederschlagsfähigen Wasserdampf enthalten,
wobei die Beobachtungsdaten und die Radiosondendaten geografisch und zeitlich einander entsprechen; und
Erzeugen eines trainierten Modells (121, 122), das die Höhe-für-Höhe-Wasserdampfdichten oder die niederschlagsfähige Wassermenge basierend auf den Trainingsdaten ausgibt, wenn Fragedaten eingegeben werden.

2. Programm (P), das einen Computer veranlasst, eine Verarbeitung auszuführen, aufweisend:
Erfassen von Beobachtungsdaten, die von einem Mikrowellenradiometer (1) gemessen werden; und
Ausgeben von Höhe-für-Höhe-Wasserdampfdichten oder eines niederschlagsfähigen Wasserdampfs durch Eingeben der Beobachtungsdaten in das trainierte Modell (121, 122), das durch das Verfahren nach Anspruch 1 erzeugt wird, das die Höhe-für-Höhe-Wasserdampfdichten oder die niederschlagsfähige Wassermenge ausgibt, wenn die Beobachtungsdaten als Fragedaten eingegeben werden.

3. Programm (P) nach Anspruch 2, wobei die Beobachtungsdaten eine Funkwellenintensität für jede von einer Vielzahl von Frequenzen enthalten.

4. Programm (P) nach Anspruch 3, wobei ein Dimensionsreduktionsprozess an der Funkwellenintensität für jede der Vielzahl von Frequenzen, die in den Beobachtungsdaten enthalten sind, durchgeführt wird, und
die Beobachtungsdaten, die die Funkwellenintensität enthalten, die dem Dimensionsreduktionsprozess unterzogen wird, in das trainierte Modell (121, 122) eingegeben werden.

5. Programm (P) nach einem der Ansprüche 2 bis 4, wobei die Trainingsdaten, die beim Erzeugen des trainierten Modells verwendet werden, ferner klimatologische Daten enthalten, die Temperatur, Feuchtigkeit, Luftdruck oder Niederschlag an einer Stelle enthalten, an der das Mikrowellenradiometer (1) installiert ist, und die Verarbeitung ferner aufweist:
Ausgeben der Höhe-für-Höhe-Wasserdampfdichten oder der niederschlagsfähigen Wassermenge durch Eingeben der Beobachtungsdaten und der klimatologischen Daten in das trainierte Modell (121, 122).

6. Programm (P) nach einem der Ansprüche 2 bis 5, wobei eine Vielzahl von Teilen der Beobachtungsdaten, die zu einer Vielzahl von Zeitpunkten von dem Mikrowellenradiometer (1) gemessen werden, erfasst werden; und die Verarbeitung ferner aufweist:
Erzeugen von Änderungsinformationen einer Höhenwasserdampfverteilung basierend auf den jeweiligen Höhe-für-Höhe-Wasserdampfdichten zu der Vielzahl von Zeitpunkten, die von dem trainierten Modell (121, 122) ausgegeben werden; und
Ausgeben der Änderungsinformationen der Höhenwasserdampfverteilung.

7. Programm (P) nach einem der Ansprüche 2 bis 6, wobei eine Vielzahl von Teilen der Beobachtungsdaten, die zu einer Vielzahl von Zeitpunkten von dem Mikrowellenradiometer (1) gemessen werden, erfasst werden; und die Verarbeitung ferner aufweist:
Erzeugen von Änderungsinformationen eines niederschlagsfähigen Wasserdampfs basierend auf dem niederschlagsfähigen Wasserdampf zu der Vielzahl von Zeitpunkten, die von dem trainierten Modell (121, 122) ausgegeben werden; und
Ausgeben der Änderungsinformationen des niederschlagsfähigen Wasserdampfs.

8. Programm (P) nach einem der Ansprüche 2 bis 7, wobei Beobachtungsdaten für jede Stelle, die von den jeweiligen Mikrowellenradiometern (1) gemessen werden, die in einer Vielzahl von Stellen installiert sind, erfasst werden; und die Verarbeitung ferner aufweist:
Ausgeben der Höhe-für-Höhe-Wasserdampfdichten oder des niederschlagsfähigen Wasserdampfs an jeder der Vielzahl von Stellen durch Eingeben der Beobachtungsdaten für jede Stelle in das trainierte Modell (121, 122).

9. Programm (P) nach Anspruch 8, wobei Karteninformationen zum Identifizieren der Vielzahl von Stellen, an denen die Vielzahl von Mikrowellenradiometern (1) installiert ist, erfasst werden; und die Verarbeitung ferner aufweist:
Überlagern der Höhe-für-Höhe-Wasserdampfdichten oder des niederschlagsfähigen Wasserdampfs an jeder der Vielzahl von Stellen in den Karteninformationen.

10. Programm (P) nach Anspruch 8 oder 9, wobei klimatologische Daten, die Temperatur, Feuchtigkeit, Luftdruck oder Niederschlag an jeder der Vielzahl von Stellen enthalten, an denen die Vielzahl von Mikrowellenradiometern (1) installiert ist, erfasst werden; und die Verarbeitung ferner aufweist:
Eingeben der Beobachtungsdaten und der klimatologischen Daten für jede der Vielzahl von Stellen in das trainierte Modell (121, 122).

11. Schätzvorrichtung, aufweisend:
eine Erfassungseinheit (111), die konfiguriert ist, um Beobachtungsdaten zu erfassen, die von einem Mikrowellenradiometer (1) gemessen werden; und
eine Schätzeinheit, die konfiguriert ist, um die Beobachtungsdaten in ein trainiertes Modell (121, 122) einzugeben, das durch das Verfahren nach Anspruch 1 erzeugt wird, und die Höhe-für-Höhe-Wasserdampfdichten oder einen niederschlagsfähigen Wasserdampf ausgibt, wenn die Beobachtung als Fragedaten eingegeben werden,
und die Höhe-für-Höhe-Wasserdampfdichten oder den niederschlagsfähigen Wasserdampf schätzt.

12. Schätzverfahren, das von einem Computer durchgeführt wird, wobei das Schätzverfahren aufweist:
Erfassen von Beobachtungsdaten, die von einem Mikrowellenradiometer (1) gemessen werden; und
Eingeben der Beobachtungsdaten als Fragedaten in ein Lernmodell (121, 122), das durch das Verfahren nach Anspruch 1 erzeugt wurde und das Höhe-für-Höhe-Wasserdampfdichten oder einen niederschlagsfähigen Wasserdampf ausgibt, wenn die Beobachtungsdaten eingegeben werden, und Schätzen der Höhe-für-Höhe-Wasserdampfdichten oder des niederschlagsfähigen Wasserdampfs.

## Revendications

1. Procédé de génération qui est réalisé par un ordinateur pour générer un modèle d'apprentissage (121, 122), comprenant :
acquérir des données d'entraînement sous forme de données de question à partir de données d'observation mesurées par un radiomètre hyperfréquence (1), les données de question incluant des densités de vapeur d'eau altitude par altitude ou une vapeur d'eau précipitable, et sous forme de données de réponse des données de sonde radio mesurées par une sonde radio, les données de réponse incluant des densités de vapeur d'eau altitude par altitude ou une vapeur d'eau précipitable, dans lequel les données d'observation et les données de sonde radio correspondent géographiquement et temporellement les unes aux autres ; et
générer un modèle entraîné (121, 122) qui délivre en sortie les densités de vapeur d'eau altitude par altitude ou la quantité d'eau précipitable en fonction des données d'entraînement lorsque des données de question sont entrées.

2. Programme (P) amenant un ordinateur à exécuter un traitement comprenant :
acquérir des données d'observation mesurées par un radiomètre hyperfréquence (1) ; et
délivrer en sortie des densités de vapeur d'eau altitude par altitude ou une vapeur d'eau précipitable en entrant les données d'observation dans le modèle entraîné (121, 122) généré par le procédé selon la revendication 1, qui délivre en sortie les densités de vapeur d'eau altitude par altitude ou la quantité de vapeur d'eau précipitable lorsque les données d'observation sont entrées en tant que données de question.

3. Programme (P) selon la revendication 2, dans lequel les données d'observation incluent une intensité d'onde radio pour chacune d'une pluralité de fréquences.

4. Programme (P) selon la revendication 3, dans lequel un processus de réduction de dimension est réalisé sur l'intensité d'onde radio pour chacune de la pluralité de fréquences incluses dans les données d'observation, et
les données d'observation incluant l'intensité d'onde radio soumise au processus de réduction de dimension sont entrées dans le modèle entraîné (121, 122) .

5. Programme (P) selon l'une des revendications 2 à 4, dans lequel les données d'entraînement utilisées lors de la génération du modèle entraîné incluent en outre des données climatologiques incluant la température, l'humidité, la pression atmosphérique ou les précipitations à un emplacement où le radiomètre hyperfréquence (1) est installé, et le traitement comprenant en outre :
délivrer en sortie les densités de vapeur d'eau altitude par altitude ou la quantité d'eau précipitable en entrant les données d'observation et les données climatologiques dans le modèle entraîné (121, 122).

6. Programme (P) selon l'une des revendications 2 à 5, dans lequel une pluralité d'éléments des données d'observation mesurées à une pluralité d'instants par le radiomètre hyperfréquence (1) sont acquis ; et le traitement comprenant en outre :
générer des informations de variation d'une répartition altitudinale de vapeur d'eau, sur la base des densités de vapeur d'eau altitude par altitude respectives à la pluralité d'instants délivrées en sortie par le modèle entraîné (121, 122) ; et
délivrer en sortie les informations de variation de la répartition altitudinale de vapeur d'eau.

7. Programme (P) selon l'une des revendications 2 à 6, dans lequel une pluralité d'éléments des données d'observation mesurées à une pluralité d'instants par le radiomètre hyperfréquence (1) sont acquis ; et le traitement comprenant en outre :
générer des informations de variation d'une vapeur d'eau précipitable en fonction de la vapeur d'eau précipitable à la pluralité d'instants délivrée en sortie par le modèle entraîné (121, 122) ; et
délivrer en sortie les informations de variation de la vapeur d'eau précipitable.

8. Programme (P) selon l'une des revendications 2 à 7, dans lequel des données d'observation sont acquises pour chaque emplacement mesuré par les radiomètres hyperfréquences (1) respectifs installés en une pluralité d'emplacements ; et le traitement comprenant en outre :
délivrer en sortie les densités de vapeur d'eau altitude par altitude ou la vapeur d'eau précipitable à chacun de la pluralité d'emplacements en entrant les données d'observation pour chaque emplacement dans le modèle entraîné (121, 122).

9. Programme (P) selon la revendication 8, dans lequel des informations cartographiques sont acquises pour identifier la pluralité d'emplacements où la pluralité de radiomètres hyperfréquences (1) sont installés ; et le traitement comprenant en outre :
superposer les densités de vapeur d'eau altitude par altitude ou la vapeur d'eau précipitable à chacun de la pluralité d'emplacements dans les informations cartographiques.

10. Programme (P) selon la revendication 8 ou 9, dans lequel des données climatologiques incluant la température, l'humidité, la pression atmosphérique ou les précipitations sont acquises à chacun de la pluralité d'emplacements où la pluralité de radiomètres hyperfréquences (1) sont installés ; et le traitement comprenant en outre :
entrer les données d'observation et les données climatologiques pour chacun de la pluralité d'emplacements dans le modèle entraîné (121, 122).

11. Dispositif d'estimation comprenant :
une unité d'acquisition (111) configurée pour acquérir des données d'observation mesurées par un radiomètre hyperfréquence (1) ; et
une unité d'estimation configurée pour entrer les données d'observation dans un modèle entraîné (121, 122) généré par le procédé selon la revendication 1, et qui délivre en sortie des densités de vapeur d'eau altitude par altitude ou une vapeur d'eau précipitable lorsque l'observation est entrée en tant que données de question, et estimer les densités de vapeur d'eau altitude par altitude ou la vapeur d'eau précipitable.

12. Procédé d'estimation qui est réalisé par un ordinateur, le procédé d'estimation comprenant :
acquérir des données d'observation mesurées par un radiomètre hyperfréquence (1) ; et
entrer les données d'observation en tant que données de question dans un modèle d'apprentissage (121, 122) qui a été généré par le procédé selon la revendication 1 et qui délivre en sortie des densités de vapeur d'eau altitude par altitude ou une vapeur d'eau précipitable lorsque les données d'observation sont entrées, et estimer les densités de vapeur d'eau altitude par altitude ou la vapeur d'eau précipitable.
